# EUROPÄISCHE PATENTANMELDUNG

(11) **EP 4 616 813 A1**
(43) Veröffentlichungstag der Anmeldung: **17.09.2025**
(21) Anmeldenummer: 24163982.2
(22) Anmeldetag: 15.03.2024
(51) Int. Cl.: A61B 17/12, A61B 17/132

(54) **BLUTGEFÄSSSTEMPEL**

(71) Anmelder: Nanos, Michael, 85276 Pfaffenhofen (DE)
(72) Erfinder: Nanos, Michael, 85276 Pfaffenhofen (DE)
(74) Vertreter: noventive Patentanwaltsgesellschaft mbH

(57) **Zusammenfassung**

Blutgefäßstempel (10) für die externale Kompression mindestens eines Blutgefäßes, insbesondere mindestens eines Blutgefäßes ausgewählt aus Vena femoralis oder Arteria femoralis, der Blutgefäßstempel (10) aufweisend eine Griffvorrichtung (18) und einen Stempel (19) aufweisend eine Stempelfläche (11).

## Beschreibung

Die Erfindung bezieht sich auf einen Blutgefäßstempel.

Im Stand der Technik ist es bekannt, dass manuell mit direktem Kontakt des Arztes zum Patienten Blutgefäße komprimiert werden können. Dies geschieht unter großer Anstrengung und meist ungenau, unsauber und ggf. unsteril. Es kann zur inadäquaten Hämostase und somit zur prolongierten Kompression kommen.

Es ist Aufgabe der Erfindung die Kompression von Blutgefäßen zu verbessern.

Die Aufgabe kann nach einem Aspekt gelöst werden durch den Blutgefäßstempel mit den Merkmalen des Anspruchs 1.

Dabei kann ein Blutgefäßstempel für die externale Kompression mindestens eines Blutgefäßes vorgesehen sein, insbesondere mindestens eines Blutgefäßes ausgewählt aus Vena femoralis oder Arteria femoralis. Der Blutgefäßstempel weist insbesondere eine Griffvorrichtung und einen Stempel auf, wobei der Stempel eine Stempelfläche aufweist. Das zu komprimierende Blutgefäß kann ausgewählt werden aus Vena femoralis oder Arteria femoralis. Ein Blutgefäß ist dabei insbesondere ein Gefäß, das für den Transport von Blut im Körper verantwortlich ist. Die Kompression bezieht sich insbesondere auf die Verringerung des Durchmessers eines Hohlorgans oder einer Struktur, indem eine Kraft auf sie ausgeübt wird, hier auf die Verengung eines Blutgefäßes, um einen Blutdurchfluss zu kontrollieren oder zu unterbinden.

Eine Kompression kann indirekt über einen Kompressions-Device in Form eines Blutgefäßstempels von außen, also insbesondere ohne invasive Schritte, erfolgen. Die Auflagefläche - auch als Stempelfläche zu bezeichnen - ermöglicht eine stabile Kompression des Gefäßes und ermöglicht gleichzeitig ein ungehindertes Arbeiten an der Punktionsstelle.

Die Femoralgefäße verlaufen parallel in der Leistenregion sowohl rechts als auch links und bestehen aus Vena femoralis und Arteria femoralis, wobei die Vena femoralis sauerstoffarmes Blut aus der unteren Extremität zum Herzen zurückführt und die Arteria femoralis sauerstoffreiches Blut vom Herzen kommend in die untere Extremität leitet.

Ein Stempel kann hier ein Werkzeug sein, das verwendet wird, um eine bestimmte Aufgabe auszuführen. In diesem Zusammenhang bezieht sich der Stempel insbesondere auf den Teil des Blutgefäßstempels, der zur Kompression verwendet wird. Der Stempel weist insbesondere mindestens eine Stempelfläche auf. Die Stempelfläche bezieht sich insbesondere auf die Oberfläche des Stempels, die zum Komprimieren des Blutgefäßes verwendet wird. In Ausführungsformen kann vorgesehen sein, dass sich zwei Auflageflächen ergeben, eine Auflagefläche, die der Stabilität dient (die größere Fläche) und eine steiler zulaufende Fläche, die zum zielgerichteten komprimieren des Gefäßes/der Gefäße dienen kann. Zusätzlich kann der Blutgefäßstempel eine Griffvorrichtung aufweisen. Es kann Ausführungsformen geben, in denen ein Griff vorgesehen sein muss.

Der Blutgefäßstempel dient insbesondere zur indirekten Kompression der Femoralgefäße bei großlumigen Kanülierungen im Rahmen von Interventionen durch die Femoralgefäße wie u.a. Herzkatheteruntersuchungen, Implantation von Impella, ECMO und weitere perkutane interventionelle- bzw. Gefäßeingriffe.

Impella bezieht sich insbesondere auf eine Marke von herzunterstützenden Pumpen, die bei Patienten mit schwerem Herzversagen verwendet werden können.

ECMO steht für Extracorporale Membranoxygenierung und bezieht sich auf einen Prozess via einem Gerät, welches extrakorporal den Gasaustausch (Sauerstoff und Kohlenstoffdioxid) durch eine künstliche Membran (Bestandteil der ECMO) gewährleisten kann. Insbesondere gleichzeitig generiert eine Zentrifugalpumpe einen kontinuierlichen Blutfluss. Somit werden Herz und/oder Lunge des Patienten unterstützt.

Hinsichtlich des Materials kann Plastik für ein "Gerüst" und Gummi für die Auflagefläche - die Stempelfläche - vorgesehen sein. Der Blutgefäßstempel kann zur einmaligen Verwendung einzeln steril verpackt sein.

Nach einem Aspekt kann die Stempelfläche ausgebildet sein, um eine externale Kompression für zwei Blutgefäße gleichzeitig auszubilden, insbesondere für die Arteria femoralis und die Vena femoralis. Der Blutgefäßstempel kann folglich in zwei Ausführungen ausgebildet sein. Die erste Ausführung (VA-Kompression) kann sowohl die Vena femoralis als auch die Arteria femoralis gleichzeitig komprimieren. In einer zweiten Ausführungsform (V/A-Kompression) kann lediglich eines der Gefäße komprimiert werden. Durch die entsprechende Ausführungsform kann jeweils ein Blutgefäß oder beide Blutgefäße komprimiert werden, die bei einer Behandlung oder einer medizinischen Anwendung komprimiert werden sollen.

Alternativ oder zusätzlich kann der Blutgefäßstempel insbesondere in einer ausgewählt von fünf Ausführungsformen vorliegen:
Die erste Ausführungsform dient insbesondere der Kompression des Gefäßpaares Vena femoralis und Arteria femoralis. Hierzu ist der Stempel ausgebildet, um in der ersten Ausführungsform 90° zum Gefäßverlauf aufgesetzt zu werden. Dabei kann eine längere Seite 90° zum Gefäßverlauf verlaufen. Der Stempel der ersten Ausführung ist ausgebildet, um mit der Auflagefläche um 90° gedreht zu werden, längs zum Gefäßverlauf, wobei entweder die Vena femoralis oder Arteria femoralis komprimiert werden kann. Es können angeschrägte Flächen vorgesehen sein, die es ermöglichen, auch in einem Winkel relativ zu der Oberfläche der Haut einen Druck aufzubringen. Der Winkel ergibt sich insbesondere zwischen der Flächennormalen der Haut in einem Aufsatzpunkt und der Flächennormalen der Auflagefläche (insbesondere der angeschrägten Fläche).

Gemäß der zweiten Ausführungsform kann der Stempel ausgebildet sein, um mit der Auflagefläche 90° zum Gefäßverlauf aufgesetzt zu werden und hierbei isoliert ein Gefäß, entweder die Vena femoralis oder die Arteria femoralis, zu komprimieren. Das Auflegen der Stempelfläche 90° zum Gefäßverlauf ermöglicht eine größere Arbeitsfläche im Bereich der Punktionsstelle.

Die dritte, vierte und fünfte Ausführungsform dient insbesondere der Kompression der Femoralgefäße. Dies kann durch eine variable Angulation der Auflagefläche auf die Gefäße erreicht werden. Hierbei kann die Auflagefläche auf unterschiedliche Weise nach unten zulaufen. In einer dritten Ausführungsform kann die Stempelfläche C-förmig gekrümmt sein. In der vierten und fünften Ausführungsform kann ein Ende im Vergleich zum anderen Ende des C-förmigen Stempels im Durchmesser parallel zu der Stempelrichtung und senkrecht zu der Stempelausdehnung (bzw. der Auflagefläche) abnehmen. Der Anwender kann die Auflagefläche abrollen und somit eine gezielte Kompression eines oder beider Gefäße in allen Gefäßachsen erreichen. Vierte und fünfte Ausführungsform können sich insbesondere dadurch unterscheiden, dass die vierte Ausführungsform eine aus mehreren Profilen zusammengepatchte Stempelfläche aufweist. Dabei können von einem spitzen Querschnitt bis zu verschiedenen trapezoiden Querschnitten Übergänge vorgesehen sein. Die fünfte Ausführungsform weist insbesondere einen kontinuierlichen Verlauf von einem flachen Stempel zu einem tiefen Stempel auf.

Nach einem Aspekt kann die Stempelfläche rechteckig ausgebildet sein. Dadurch kann eine Definition der Form vorgesehen sein, die eine Kompression der Blutgefäße ermöglicht, die für eine Anwendung vorgesehen ist.

Nach einem Aspekt kann die Stempelfläche rechteckig ausgebildet sein, mit einer ersten Seitenlänge b1 und einer zweiten Seitenlänge b2, wobei der Stempel einen rechteckigen Umfang aufweist mit einer ersten Seitenlänge B1 und einer zweiten Seitenlänge B2. Dabei kann die erste Seitenlänge b1 parallel zur ersten Seitenlänge B1 stehen. Dabei kann die zweite Seitenlänge b2 parallel zur zweiten Seitenlänge B2 stehen. Die Seitenlängen b1, b2 können dabei den beiden Seiten der Stempelfläche entsprechen. Die Seitenlängen B1, B2 können dabei den beiden Seiten einer Umfangkante einer Fläche an der Oberseite des Stempels entsprechen. Dadurch kann der rechteckige Umfang des Stempels einer rechteckigen Form der Stempelfläche zugeordnet werden, miteinander entsprechenden (proportionalen) Seitenzuordnungen, um einem Anwender eine mögliche Orientierung des Stempels und der damit verbundenen Stempelfläche aufzuzeigen, ohne dabei den Stempel umdrehen zu müssen.

Nach einem ersten Aspekt kann die erste Seitenlänge b1 kleiner sein als die erste Seitenlänge B1. Alternativ oder zusätzlich kann die zweite Seitenlänge b2 kleiner sein als die zweite Seitenlänge B2. In einer Ausführungsform können die einander entsprechenden Seitenlängen zueinander proportional sein. Dadurch kann der rechteckige Umfang des Stempels einer rechteckigen Form der Stempelfläche zugeordnet werden, mit einander entsprechenden (proportionalen) Seitenzuordnungen, um einem Anwender eine mögliche Orientierung des Stempels und der damit verbundenen Stempelfläche aufzuzeigen, ohne dabei den Stempel umdrehen zu müssen.

Nach einem Aspekt kann die Stempelfläche und die Umfangkante einer Fläche einer Oberseite des Stempels über mindestens eine abfallende Fläche verbunden sein. Dadurch kann ein gradueller Übergang zwischen der Umfangkante und der Stempelfläche ausgebildet sein, um etwaige Druckmarken am Patienten bei einem verkanteten Aufsetzen zu reduzieren oder zu verhindern.

Die Oberseite des Stempels entspricht dabei insbesondere der Seite, die einer Greifvorrichtung, wie etwa einem Griff zugewandt ist. Die Griffvorrichtung bzw. der Griff kann in die Oberseite münden.

Nach einem Aspekt kann mindestens eine der ersten Seitenlänge b1 der Auflagefläche oder der zweiten Seitenlänge b2 der Auflagefläche mindestens einer der ersten Seitenlänge B1 des Stempels oder der zweiten Seitenlänge B2 des Stempels in der Länge entsprechen. Dadurch kann sich die Stempelfläche in einer Richtung parallel zu einer Seite des Stempels komplett über diese Seite erstrecken. In anderen Ausführungsformen kann eine erste Seitenlänge b1 der Auflagefläche und eine zweite Seitenlänge b2 der Auflagefläche kürzer sein als eine erste Seitenlänge B1 des Stempels und eine zweite Seitenlänge B2 des Stempels.

Nach einem Aspekt kann die Stempelfläche eine Krümmung aufweisen. Dadurch kann auch ein verkipptes Aufsetzen auf einer zu komprimierenden Stelle mit mindestens einem Blutgefäß erfolgen. Dadurch kann auch eine vorteilhafte Positionierung eines Anwenders zu einem Patienten eingenommen werden, da keine senkrechte Kompression erfolgen muss.

Die Krümmung kann dabei insbesondere derart ausgebildet sein, dass die Krümmung an einer Unterseite, die der Oberseite, wie sie an anderer Stelle hierin beschrieben ist, angeordnet ist. Dadurch kann insbesondere der Stempel eine größte Dicke an einer Stelle aufweisen, die unter der Griffvorrichtung angeordnet ist.

Nach einem Aspekt kann die Stempelfläche aus mindestens zwei Profilen zusammengepatcht sein. Dadurch können verschiedene Stempelflächen für verschiedene Aufsatzanwendungen vorgesehen sein. Dadurch kann ein Blutgefäßstempel für verschiedene Einsätze bei einer Behandlung vorgesehen sein. Die Stempelfläche kann mindestens zwei Profile, weiter insbesondere mindestens drei Profile, weiter insbesondere maximal fünf Profile aufweisen.

Der Begriff zusammengepatcht bezieht sich dabei insbesondere darauf, dass die Stempelfläche mehrere einander sich berührende Stempelflächen aufweist, die etwa in einem Winkel an einer Unterseite eines Stempels angeordnet sein können.

Eine Ausführungsform des Blutgefäßstempels kann sein, dass die Stempelfläche aus mindestens zwei Profilen zusammengepatcht ist. Dabei handelt es sich insbesondere um verschiedene geometrische Formen oder Strukturen auf der Stempelfläche, bzw. in Bezug auf den Umfang der Stempelfläche, die für unterschiedliche Anwendungszwecke vorgesehen sein kann. Die Stempelfläche kann mindestens zwei Profile, weiter insbesondere mindestens drei Profile, weiter insbesondere maximal fünf Profile aufweisen.

Der Begriff Profil bezieht sich hierbei insbesondere auf eine derartige Struktur oder Form auf der Stempelfläche. Diese Profile können unterschiedlich geformt sein, um verschiedene Kompressionseffekte zu erzielen,je nachdem welche Art von Blutgefäß komprimiert werden soll. Dabei kann sich der Begriff Profil insbesondere auch auf eine dreidimensionale Struktur beziehen, die der Stempel ausbilden kann. Auf dieser dreidimensionalen Struktur kann die Fläche verlaufen, die für ein Auflegen vorgesehen ist.

Die Stempelfläche ist insbesondere die Oberfläche des Stempels, die zum Komprimieren des Blutgefäßes verwendet wird. Diese kann an einer Unterseite des Stempels angeordnet sein. Durch das zusammenpatchen verschiedener Profile auf der Stempelfläche können flexible und anpassbare Kompressionseffekte erzielt werden, die für unterschiedliche medizinische Anwendungen geeignet sein können.

Der Term "zusammengepatcht" bezieht sich hierbei insbesondere auf die Verbindung oder das Zusammenfügen verschiedener Profile auf der Stempelfläche, um variablere Kompressionseffekte mit einem Stempel zu ermöglichen.

Nach einem Aspekt kann der Stempel an mindestens einem Umfangbereich flach ausgebildet sein. Dabei kann die Stempelfläche mit einer oberen Fläche zusammentreffen. Alternativ oder zusätzlich kann dabei der Stempel an mindestens einem Umfangbereich dick ausgebildet sein, insbesondere derart, dass der Umlaufbereich über einer Stempelfläche angeordnet ist. Dabei kann eine Verbindung im Wesentlichen senkrecht zu einer Druckrichtung verlaufen.

Eine Ausführungsform des Blutgefäßstempels kann sein, dass der Stempel an mindestens einem Umfangbereich flach ausgebildet sein kann und dabei die Stempelfläche mit einer oberen Fläche zusammentreffen kann. Die obere Fläche des Stempels ist insbesondere einer Griffvorrichtung zugewandt und kann als Auflagefläche für die Kompression verwendet werden. Der Umlaufbereich der oberen Seite des Stempels ist der Teil, der um den Stempel herumläuft.

Der Stempel kann an mindestens einem Umfangbereich dick ausgebildet sein, insbesondere in einer Weise, dass der Umlaufbereich über einer Stempelfläche angeordnet ist. Die Verbindung zwischen dem Umlaufbereich und der Stempelfläche verläuft im Wesentlichen senkrecht zu einer Druckrichtung. Diese Anordnung ermöglicht eine stabile Kompression des Blutgefäßes.

Nach einem Aspekt kann der Stempel an mindestens einem Umfangsbereich schmal ausgebildet sein, insbesondere derart, dass sich eine vordere und eine hintere Umfangsseite treffen, insbesondere ohne einen seitlichen Umfangseitenbereich. Dadurch kann der Stempel eine Spitze aufweisen, die es insbesondere ermöglicht auch eine punktuelle Kompression ausüben zu können. Dadurch kann auch in speziellen Anwendungen zielgenau ein Druck auf ein Blutgefäß ausgeübt werden.

Nach einem Aspekt kann mindestens eine der Seitenlängen b1, b2, b4 zwischen 5 mm und 50 mm, insbesondere zwischen 10 mm und 20 mm, weiter insbesondere zwischen 12 mm und 18 mm sein. Darüber hinaus kann mindestens eine der Seitenlängen B1, B2 zwischen 20 mm und 100 mm, insbesondere zwischen 30 mm und 60 mm, weiter insbesondere zwischen 40 mm und 50 mm oder sogar zwischen 60 mm und 100 mm, insbesondere zwischen 70 mm und 90 mm, weiter insbesondere zwischen 75 mm und 85 mm sein. Diese spezifizierten Größenangaben können darauf abzielen, eine optimale indirekte Kompression der Femoralgefäße zu erreichen.

Weitere Ausführungsformen, Fortbildungen bzw. Ausprägungen werden durch die abhängigen Ansprüche beschrieben. Weitere Vorteile, Merkmale und Einzelheiten der Erfindung ergeben sich aus der nachfolgenden Beschreibung, in der unter Bezugnahme auf die Zeichnungen Ausführungsbeispiele der Erfindung im Einzelnen beschrieben sind. Dabei können die in den Ansprüchen und in der Beschreibung erwähnten Merkmale jeweils einzeln für sich oder in beliebiger Kombination erfindungswesentlich sein. Es zeigen schematisch:
Fig. 1A eine Unterseitenansicht einer ersten Ausführungsform des Blutgefäßstempels;
Fig. 1B eine Oberseitenansicht einer ersten Ausführungsform des Blutgefäßstempels;
Fig. 1C eine Seitenansicht einer ersten Ausführungsform des Blutgefäßstempels;
Fig. 2A eine Unterseitenansicht einer zweiten Ausführungsform des Blutgefäßstempels;
Fig. 2B eine Oberseitenansicht einer zweiten Ausführungsform des Blutgefäßstempels;
Fig. 2C eine Seitenansicht einer zweiten Ausführungsform des Blutgefäßstempels;
Fig. 3A eine Seitenansicht einer dritten Ausführungsform des Blutgefäßstempels;
Fig. 3B eine Ansicht einer vierten Ausführungsform des Blutgefäßstempels;
Fig. 3C eine Ansicht einer fünften Ausführungsform des Blutgefäßstempels; und
Fig. 3D Schnittansichten von Stempelprofilen.

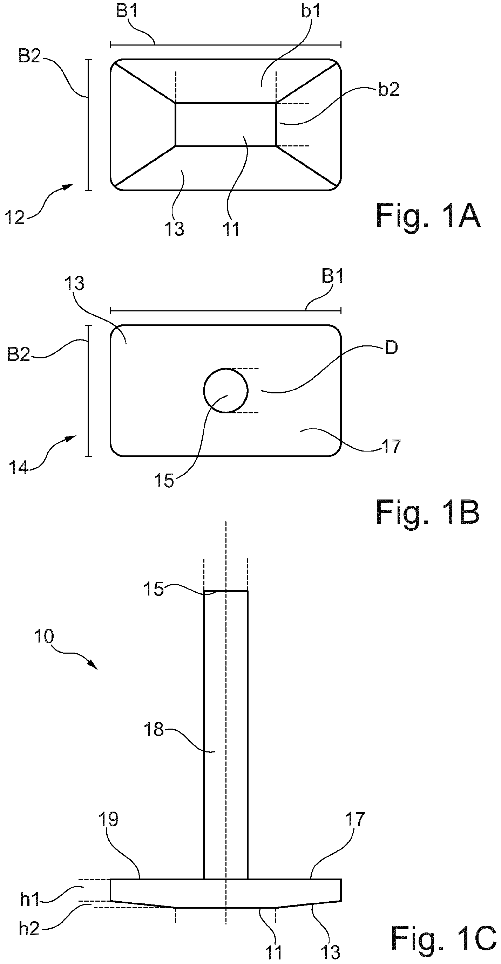

Fig. 1A zeigt eine Unterseitenansicht 12 einer exemplarischen ersten Ausführungsform eines rechteckigen Blutgefäßstempels 10. Der Blutgefäßstempel ist dabei in Fig. 1C in einer Seitenansicht gezeigt. Dieser weist einen Griff 18 auf, der an einer Oberseite 17 des Stempels 19 mittig angeordnet ist, wie in der Fig. 1B gezeigt. Der Stempel weist einen rechteckigen Umfang mit Seitenlängen B1, B2 auf, der hier bei 80 mm für B1 und 45 mm für B2 angegeben ist. Die Ecken des Blutgefäßstempels 10 können abgerundet sein.

Die Stempelfläche 11 ist insbesondere ebenfalls rechteckig ausgebildet, mit Seitenlängen b1, b2, wobei die lange Seite b1 der Stempelfläche der langen Seitenlänge B1 des Stempels zugeordnet sein kann, um dadurch eine Orientierung der Stempelfläche bei einer Oberseitenansicht 14, wie sie in Fig. 1B dargestellt ist erkennen zu können. Die Dicke des Griffs (entsprechend dem Durchmesser der Oberseitenfläche 15) kann dabei der kurzen Seitenlänge b2 der Stempelfläche 11 entsprechen. Die Seitenlängen b1, b2 der Stempelfläche 11 kann dabei proportional zu den Seitenlängen B1, B2 des Stempels sein. Die Seitenlänge b1 der Stempelfläche 11 kann 35 mm aufweisen, insbesondere +/- 20 mm. Die Seitenlänge b2 der Stempelfläche 11 kann 15 mm aufweisen, insbesondere +/- 10 mm. Die Seitenlänge B1 kann dabei 80 mm +/- 20 mm betragen. Die Seitenlänge B2 kann 45 mm +/-15 mm betragen.

Wie in Fig. 1C gezeigt kann der Blutgefäßstempel 10 einen Griff 18 aufweisen, der an der Oberseitenfläche 17 angeordnet ist. Der Stempel weist eine Höhe h1 auf, hier exemplarisch von 8mm. Alternativ kann diese auch abweichen und eine Dicke von bis zu 15 mm aufweisen. Von einer unteren Kante des Stempels 19 ist insbesondere eine schräge Fläche 13 angeordnet, um einen Übergang in die Stempelfläche 11 vermitteln zu können. Dabei kann die schräge Fläche 13 über eine Höhe h2 absinken, hier exemplarisch von 2 mm. Alternativ können Abweichungen von bis zu 10 mm möglich sein. Der Griff 18 kann eine Länge von 100 mm aufweisen. Alternativ kann der Griff 18 eine Länge von bis zu 150 mm aufweisen. Der Griff kann einen Durchmesser D aufweisen. Dieser entspricht hier der Seitenlänge b2, was nicht immer der Fall ist, sondern dieser kann auch +/- 10 mm um den hier angegebenen Wert für die Seitenlänge b2 schwanken. Alternativ oder zusätzlich kann der Durchmesser D dem Wert der Seitenlänge b1 entsprechen, was nicht immer der Fall ist, sondern dieser kann auch +/- 10 mm um den hier angegebenen Wert für die Seitenlänge b1 schwanken.

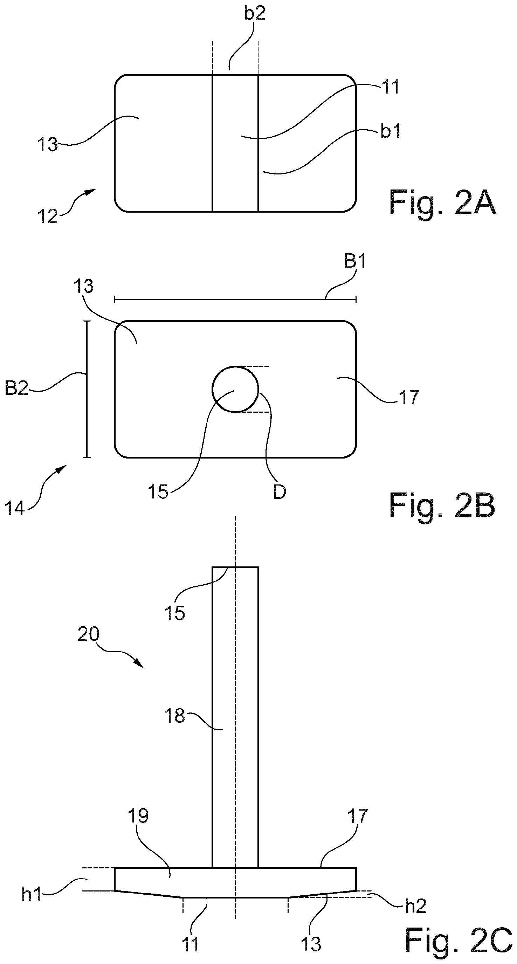

Die erste exemplarische Ausführungsform des Blutgefäßstempels 10 ist dabei insbesondere eine VA-Ausführungsform, die insbesondere ausgebildet ist, um sowohl die Vena femoralis und die Arteria femoralis zu komprimieren, da sie eine Stempelfläche 11 aufweist, deren Bemessung dem durchschnittlichen Abstand der beiden in einem Durchschnittspatienten entspricht. Fig. 2A zeigt eine Unterseitenansicht 12 einer zweiten exemplarischen Ausführungsform des Blutgefäßstempels 20. Dabei entsprechen die Ausbildung und Merkmale weitestgehend der Beschreibung zur ersten exemplarischen Ausführungsform des Blutgefäßstempels 10, wie er in den Figs. 1A bis 1C dargestellt und diesbezüglich beschrieben ist. Fig. 2B zeigt eine Oberseitenansicht 14 einer zweiten Ausführungsform des Blutgefäßstempels 20. Fig. 2C zeigt eine Seitenansicht einer zweiten Ausführungsform des Blutgefäßstempels 20.

Ein möglicher Unterschied zwischen den beiden Ausführungsformen ist dabei insbesondere die Stempelfläche 11, die wie in der Fig. 2A gezeigt zwar ebenfalls eine rechteckige Form aufweisen kann, deren Seitenlänge b2 indes der Seitenlänge B2 des Stempels 20 entsprechen kann. Darüber hinaus gibt es insbesondere eine abfallende Fläche nur in einer Richtung von einer Umlaufkante zu dem Bereich, in dem die Auflagefläche angeordnet sein kann. Alternativ oder zusätzlich kann die Stempelfläche im Wesentlichen, im Unterschied zur ersten Ausführungsform, um 90 Grad gedreht sein. Damit können insbesondere die Seitenlängen b1 und b2 "die Rolle tauschen". Dabei kann die Seitenlänge b1 der Seitenlänge B2 des Stempels von 45 mm +/- 15 mm entsprechen. Die Seitenlänge b2 kann dabei wie zuvor in Bezug auf die Fig. 1A beschrieben sein.

Die zweite exemplarische Ausführungsform des Blutgefäßstempels 20 ist dabei insbesondere eine V/A-Ausführungsform, die insbesondere ausgebildet ist, um entweder die Vena femoralis oder die Arteria femoralis zu komprimieren, da sie eine Stempelfläche 11 aufweist, deren Bemessung unterhalb des durchschnittlichen Abstands der beiden in einem Durchschnittspatienten entspricht. Dadurch kann lediglich eine der beiden Gefäße komprimiert werden. Die Höhe der Stempelseite h1 kann dabei zwischen 2 und 8 mm sein. Alternativ kann sie bis zu 15 mm aufweisen. Die Höhe der abfallenden Fläche h2 (also wie weit die abfallende Fläche und damit die Auflagefläche von der Seite vorsteht) kann 2 bis zu 12 mm betragen.

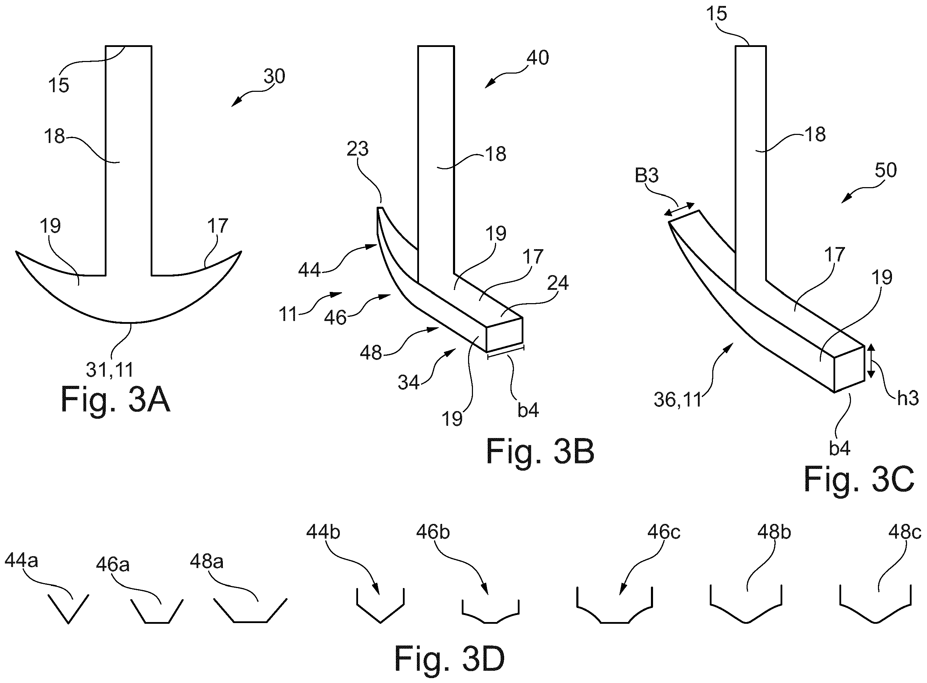

Fig. 3A zeigt eine Seitenansicht einer dritten exemplarischen Ausführungsform des Blutgefäßstempels 30. Dabei ist insbesondere eine Krümmung 31 einer gekrümmten Stempelfläche 11 vorgesehen. Dadurch kann eine Angulation des Blutgefäßstempels ermöglicht werden, über einen Winkelbereich der Krümmung 31.

Fig. 3B zeigt eine Ansicht einer vierten exemplarischen Ausführungsform des Blutgefäßstempels 40. Dabei kann die Stempelfläche 11 mehrere Profile 36 aufweisen, insbesondere ein erstes Profil 44, ein zweites Profil 46 und ein drittes Profil 48.

Fig. 3D zeigt Schnittansichten exemplarischer Profile 36. Die Profile sind dabei eine Schnittansicht parallel zu einer kürzesten Seitenlänge B2 des Stempels 19. Dabei kann insbesondere ein spitzes Profil 44a ausgebildet sein. Alternativ oder zusätzlich kann ein Spitzprofil mit Seitenwänden 44b vorgesehen sein. Alternativ oder zusätzlich kann ein Profil 46a mit geschnittener Spitze vorgesehen sein. Alternativ oder zusätzlich kann ein Profil 46b mit geschnittener Spitze und Seitenwänden vorgesehen sein. Alternativ oder zusätzlich kann ein Profil 46c mit geschnittener Spitze und

Seitenwänden vorgesehen sein. Alternativ oder zusätzlich kann ein Profil 48a mit geschnittener Spitze vorgesehen sein. Alternativ oder zusätzlich kann ein Rundprofil 48b mit einer ersten Rundung vorgesehen sein. Alternativ oder zusätzlich kann ein Rundprofil 48c mit einer zweiten Rundung vorgesehen sein. Dabei können alle denkbaren Kombinationen von verschiedenen Profilen 36 vorgesehen sein.

Dabei kann eine Spitze 23 an einem Stempel 19 vorgesehen sein, um eine punktgenaue Kompression zu ermöglichen. Diese kann auch als schmale Kante des Stempels 19 bezeichnet werden. An einem anderen Ende des Stempels 19 kann eine breite Kante 24 vorgesehen sein, mit einer Breite b4, hier zwischen 0,5 cm und 3 cm, insbesondere von genau 2 cm. Auch die Höhe h3 kann hier, entsprechend wie in Fig. 3C dargestellt, zwischen 1 cm und 3 cm liegen, insbesondere bei genau 2 cm.

Fig. 3C zeigt eine Ansicht einer fünften exemplarischen Ausführungsform des Blutgefäßstempels 50. Dabei kann ein Profil 36 eine Krümmung 31 aufweisen. Dabei kann eine flache Kante vorgesehen sein, bei der die Oberseite 17 direkt in die Stempelfläche 11 übergeht. Die flache Kante kann eine Breite B3 zwischen 0,5 und 3 cm aufweisen. Auf der anderen Seite kann eine tiefe (oder auch dicke) Kante vorgesehen sein, bei der eine Höhe h3 zwischen 1 cm und 3 cm, insbesondere von genau 2 cm vorgesehen sein kann.

### BEZUGSZEICHENLISTE

- 10: erste Ausführungsform eines rechteckigen Blutgefäßstempel
- 11: Stempelfläche
- 12: Unterseitenansicht eines Blutgefäßstempels
- 13: abfallende Fläche
- 14: Oberseitenansicht eines Blutgefäßstempels
- 15: Griffoberseite
- 17: Stempeloberseite
- 18: Griff
- 19: Stempel
- 20: zweite Ausführungsform eines rechteckigen Blutgefäßstempel
- 23: Spitze
- 24: breite Stelle
- 30: dritte Ausführungsform eines Blutgefäßstempels mit Krümmung
- 31: Krümmung einer gekrümmten Stempelfläche
- 36: Profil
- 40: vierte Ausführungsform eines Blutgefäßstempels
- 44: erstes Profil
- 44a: Spitzprofil
- 44b: Spitzprofil mit Seitenwänden
- 46a: Profil mit geschnittener Spitze
- 46b: Profil mit geschnittener Spitze und Seitenwänden
- 46c: Profil mit geschnittener Spitze und Seitenwänden
- 48a: Profil mit geschnittener Spitze
- 48b: Rundprofil mit einer ersten Rundung
- 48c: Rundprofil mit einer zweiten Rundung
- 46: zweites Profil
- 48: drittes Profil
- 50: fünfte Ausführungsform eines Blutgefäßstempels
- b1, b2, b4: Seitenlänge der Stempelfläche
- B1, B2: Seitenlänge des Stempels
- h1: Höhe der Stempelseite
- h2: Höhe der abfallenden Fläche

## Patentansprüche

1. Blutgefäßstempel (10) für die externale Kompression mindestens eines Blutgefäßes, insbesondere mindestens eines Blutgefäßes ausgewählt aus Vena femoralis oder Arteria femoralis, der Blutgefäßstempel (10) aufweisend eine Griffvorrichtung (18) und einen Stempel (19) aufweisend eine Stempelfläche (11).

2. Blutgefäßstempel (10) nach Anspruch 1, **dadurch gekennzeichnet, dass** die Stempelfläche (11) ausgebildet ist, um eine externale Kompression für zwei Blutgefäße gleichzeitig auszubilden, insbesondere für die Arteria femoralis und die Vena femoralis.

3. Blutgefäßstempel (10) nach einem der Ansprüche 1 oder 2, **dadurch gekennzeichnet, dass** die Stempelfläche (11) rechteckig ausgebildet ist.

4. Blutgefäßstempel (10) nach einem der voranstehenden Ansprüche, **dadurch gekennzeichnet, dass** die Stempelfläche (11) rechteckig ausgebildet ist, mit einer ersten Seitenlänge b1 und einer zweiten Seitenlänge b2, wobei der Stempel (19) einen rechteckigen Umfang aufweist mit einer ersten Seitenlänge B1 und einer zweiten Seitenlänge B2, wobei die erste Seitenlänge b1 parallel zur ersten Seitenlänge B1 steht und wobei die zweite Seitenlänge b2 parallel zur zweiten Seitenlänge B2 steht.

5. Blutgefäßstempel (10) nach einem der voranstehenden Ansprüche, insbesondere nach Anspruch 4, **dadurch gekennzeichnet, dass** die erste Seitenlänge b1 kleiner ist als die erste Seitenlänge B1 und/oder wobei die zweite Seitenlänge b2 kleiner ist als die zweite Seitenlänge B2.

6. Blutgefäßstempel (10) nach einem der voranstehenden Ansprüche, insbesondere nach Anspruch 5, **dadurch gekennzeichnet, dass** die Stempelfläche (11) und die Umfangkante einer oberen Fläche (17) des Stempels (19) über mindestens eine abfallende Fläche (13) verbunden sind.

7. Blutgefäßstempel (10) nach einem der voranstehenden Ansprüche, **dadurch gekennzeichnet, dass** mindestens eine der ersten Seitenlänge b1 oder der zweiten Seitenlänge b2 der ersten Seitenlänge B1 oder der zweiten Seitenlänge B2 in der Länge entspricht.

8. Blutgefäßstempel (10) nach einem der voranstehenden Ansprüche, **dadurch gekennzeichnet, dass** die Stempelfläche (11) eine Krümmung (31) aufweist.

9. Blutgefäßstempel (10) nach einem der voranstehenden Ansprüche, **dadurch gekennzeichnet, dass** die Stempelfläche (11) aus mindestens zwei Profilen (44, 46, 48) zusammengepatcht ist.

10. Blutgefäßstempel (10) nach einem der voranstehenden Ansprüche, **dadurch gekennzeichnet, dass** der Stempel (19) an mindestens einem Umfangbereich flach ausgebildet ist, wobei die Stempelfläche (11) mit einer oberen Fläche (17) zusammentrifft und/oder wobei der Stempel (19) an mindestens einem Umfangbereich dick ausgebildet ist, derart, dass der Umlaufbereich über einer Stempelfläche (11) angeordnet ist, wobei eine Verbindung ausgerichtet ist, um im Wesentlichen senkrecht zu einer Druckrichtung zu verlaufen.

11. Blutgefäßstempel (10) nach einem der voranstehenden Ansprüche, **dadurch gekennzeichnet, dass** der Stempel (19) an mindestens einem Umfangbereich schmal ausgebildet ist, derart, dass sich eine vordere und eine hintere Umfangsseite treffen, insbesondere ohne einen seitlichen Umfangseitenbereich.

12. Blutgefäßstempel (10) nach einem der voranstehenden Ansprüche, **dadurch gekennzeichnet, dass** mindestens eine der Seitenlängen b1, b2, b4 zwischen 5 mm und 50 mm ist, weiter insbesondere zwischen 10 mm und 20 mm, weiter insbesondere zwischen 12 mm und 18 mm ist; und/oder wobei mindestens eine der Seitenlängen B1, B2 zwischen 20 mm und 100 mm ist, insbesondere zwischen 30 mm und 60 mm, weiter insbesondere zwischen 40 mm und 50 mm ist; und/oder wobei mindestens eine der Seitenlängen B1, B2 zwischen 60 mm und 100 mm ist, insbesondere zwischen 70 mm und 90 mm, weiter insbesondere zwischen 75 mm und 85 mm ist.
